Europäisches Patentamt

European Patent Office

Office européen des brevets

(11) Publication number: **0 277 237**
**A1**

# EUROPEAN PATENT APPLICATION

published in accordance with Art. 158(3) EPC

(21) Application number: **87904554.0**

(22) Date of filing: **09.07.87**

Data of the international application taken as a basis:

(86) International application number:
**PCT/JP87/00486**

(87) International publication number:
**WO88/00618 (28.01.88 88/03)**

(51) Int. Cl.³: **C 12 Q 1/68**
**C 12 N 15/00**
**//C12Q1/04**

(30) Priority: **10.07.86 JP 162394/86**

(43) Date of publication of application:
**10.08.88 Bulletin 88/32**

(84) Designated Contracting States:
**BE CH DE FR GB IT LI NL SE**

(71) Applicant: **TORAY INDUSTRIES, INC.**
**2-1, Nihonbashi Muromachi 2-chome Chuo-ku**
**Tokyo 103(JP)**

(72) Inventor: **UCHIDA, Takafumi**
**25-3-706, Ohi 1-chome Shinagawa-ku**
**Tokyo 140(JP)**

(72) Inventor: **HOSAKA, Shuntaro**
**3865, Jindaiji Mitaka-shi**
**Tokyo 181(JP)**

(72) Inventor: **MIURA, Kumiko**
**3-5-18, Daigiri Fujisawa-shi**
**Kanagawa 251(JP)**

(74) Representative: **Kador & Partner**
**Corneliusstrasse 15**
**D-8000 München 5(DE)**

(54) METHOD OF INSPECTING SPECIMEN FOR BACTERIA AND PROBE THEREFOR.

(57) A method of inspecting specimen for bacteria and probe used therefor. The method comprises inspecting specimen for bacteria using a probe prepared by labelling DNA or RNA containing a base sequence complementary to a base sequence of at least 12 consecutive bases of ribosomal RNA of *Escherichia coli* with a labelling substance. This method enables almost all of bacteria to be detected comprehensively and classified into gram-negative and gram-positive bacteria.

SPECIFICATION

Method of Assaying Bacteria and Probe Used Therefor

TECHNICAL FIELD

This invention relates to a method for assaying bacteria in a testing sample derived from living organisms such as human, animals and plants, and to a probe used for the method.

BACKGROUND ART

In general, in cases where a patient gets a high fever, where the number of leucocytes is increased, or where the number of abnormal leucocytes (undifferentiated leucocytes and leucocytes having intoxicating granules) is increased, bacterial infection is suspected. However, high fever and increase in the number of leucocytes may be caused by another factor. Needless to say, therapeutic method varies depending on the etiological cause, so that it is important to determine whether the patient is infected by bacteria or not.

One of the important testing samples is blood. Representative diseases in which assay of bacteria in the blood is required are bacteremia and septicemia. Bacteremia is a syndrome in which bacteria exist in the blood, and the situation wherein the bacteria are transiently contained in the blood is also included in the bacteremia. Septicemia is a series of clinical images resulting from the fact that a nidus is formed in the body and pathogenic bacteria are continuously or periodically released from the nidus. Septicemia is an infectious disease accompanying severe clinical symptoms, which is difficult to cure, and usually shows acute progress. In general, septicemia is brought about subsequent to a basic disorder such as malignant tumor, blood disorder and hepatocholangitic disorder. Bacteremia is a symptom wherein bacteria enter the blood at a specific peroid in a circumscribed infectious disease such as typhoid, paratyphoid, brucellosis, jackrabbit disease, anthrax, pneumenia, meningitis, cholecystitis and nephropyelitis, and the bacteria are scattered in the whole body. Bacteremia may progress to septicemia. When the existence of bacteria in the blood is suspected, the patient is often in a serious condition, so that the detection of bacteria is indispensable to an appropriate diagnosis and therapy.

Detection of bacteria in the blood and piercing fluid is currently conducted by culture-assay method (aerobic and anaerobic culture). That is, the existence of bacteria is evaluated after culturing a mixture of aseptically collected blood and an aerobic or an anaerobic culture liquid medium in a culture bottle at $37^{O}$C for several days. Although there is a method in which the number of bacteria is counted after a culture on an agar medium, this method is now scarcely employed. Recently, antibody to a bacterium is prepared and it is tried to detect the bacterium by an antigen-antibody reaction.

Another important testing sample for which an assay of bacteria is required is urine. In infectious diseases of urinary tract such as nephropyelitis and urocystitis, the target of the assay is the bacteria in the urine. Although bacteria exist in the urine of normal humans, if the bacteria population is $10^5$/ml or more, it is evaluated that the urinary tract is infected, and if the bacteria population is $10^3$/ml or less, it is evaluated to be normal. If the bacteria population is therebetween, i.e., $10^3$/ml to $10^5$/ml, further detailed examination is conducted to evaluate the existence of infection. Although culturing method is the standard method for the determination of bacteria population, there are chemical assaying methods in which the population of $10^5$/ml or more is evaluated to be positive, which includes nitrite method (Griess test), glucose oxidase method, tetrazolium-reduction method, catalase method, ATP

mesuring method and Nitrite-Esterase method.
Identification of bacteria is conducted by
differentiation culture after conducting separation
culture.

Other important samples for which assay of bacteria
is required include feces, pus, phlegm, abrasion product
of mucosa, foods and drinks such as milk and meat. The
bacteria in these samples are also assayed by culturing
method in most cases.

Assaying bacteria in the blood by the conventional
culturing method has the following 5 problems. The first
problem is that the method cannot detect dead bacteria.
However, since the bacteria forms a nidus at a restricted
location in the body, live bacteria which can be detected
by the culturing method are scarecely released to the
blood. Further, since phagocytes such as neutrophils,
monocytes and macrophages gather in the nidus, most of
the bacteria in the blood are dead bacteria or eaten
bacteria by the phagocytes. This means that the bacteria
in the blood or in the nidus in the body may not be
detected by the conventional culturing method, and a
positive sample may be evaluated as negative. If dead
bacteria can be detected, the possible existence of the
live bacteria can be predicted, so that it is a great
problem that the dead bacteria cannot be detected. The
second problem, which is basically the same as the first
problem, is that the assay cannot be conducted after a
chemotherapy such as administration of antibiotics. This
is because that the bacteria in the blood are killed by
the administration of the chemotherapuetic and so the
bacteria are apparently not detected by the culturing
method.

The third problem is that it takes a long time to
obtain the results. By the culturing method, it usually
takes several days to obtain the results. However, the
patient subjected to the assay is often in a serious condition, so that an early treatment is required. Thus, it is important in the therapy to shorten the time required for obtaining the results. The fourth problem is that not all kinds of bacteria can be detected by the culturing method because only 2 kinds of culture media (culture media for aerobic bacteria and anaerobic bacteria) are used while suitable culture medium varies depending on the kind of bacteria to be cultured. The fifth problem is that the bacteria population cannot be determined by the culturing method.

In order to improve these drawbacks, antibody specific to the bacteria is prepared and it is tried to detect the bacteria by an antigen-antibody reaction. However, it is difficult to prepare an antibody which is common to various kinds of bacteria, so that this method is not employed in practice.

The object of the invention is to solve the above-mentioned problems of the conventional method and to quantitatively detect almost all kinds of dead bacteria and bacteria eaten by phagocytes in the blood in a short time.

As for the samples other than the blood, conventional assay of bacteria which entirely relies on the culture of bacteria has the similar problems. Phagocytosis by the phagocytes may be slighter than in the blood, the problem that it is difficult to detect bacteria by culturing the same after administration of chemotherapeutics such as antibiotics is common to any of the samples. Further, in cases where the identification of the bacterial species is made by culturing alone, a number of culturing is required to obtain the result.

The object of the present invention is to shorten the time for assaying the bacteria by quickly detecting or identifying the bacteria without culturing the bacteria, or by identifying the bacteria without culturing the bacteria even if a culture is employed for growing or separating the bacteria.

## DISCLOSURE OF THE INVENTION

This invention is based on the discovery that a DNA or RNA probe having a base sequence complementary to that of a ribosomal RNA of E. coli hybridizes with the ribosomal RNAs of bacteria other than E. coli (DNA probe forms a DNA-RNA double strand and RNA probe forms a RNA-RNA double strand), and does not hybridize with the ribosomal RNAs of human. The constitution of this invention is as follows:

That is, this invention provides a method of assaying bacteria in a sample by using a probe prepared by labelling a DNA or RNA with a labelling substance, which DNA or RNA contains a base sequence complementary to a base sequence of a ribosomal RNA of E. coli, the latter base sequence containing at least 12 successive bases.

## BEST MODE FOR CARRYING OUT THE INVENTION

As the DNA or RNA containing a base sequence complementary to a base sequence of a ribosomal RNA of E. coli which contains at least 12 successive bases (hereinafter referred to as complementary base sequence for short), any base sequence which hybridizes with the ribosomal RNA of E. coli and which does not hybridize with the human ribosomal RNA may be employed.

The whole base sequence of the ribosomal RNA of E. coli is described, for example, by J. Brosius et al., (J. Mol. Biol. 148, 107-127 (1981)), and the total base number is about 7,500. As the DNA containing the complementary base sequence, fragments obtained by cutting the chromosomal DNA of E. coli with an appropriate restriction enzyme, as well as those chemically synthesized may be used. Although the DNA containing the complementary base sequence thus prepared may be used as it is, the DNA may be used after recombining with a plasmid (such as pBR322, pUC18 and

pUC19) or with a phage (M13). The complementary base sequence may contain any number of bases as long as it is not less than 12, and the base sequence complementary to the whole base sequence of E. coli ribosomal RNA may be used. Since a large amount of labelling substance is needed to be introduced into the probe to detect the bacteria with high sensitivity, the length of the chain is preferably not less than 50 bases, and usually those containing up to 15 kilobases are used.

By the present invention, grouping of Gram-negative bacteria and Gram-positive bacteria may be conducted. That is, the grouping of the bacteria may be made by properly using the base sequence of 5'GTTTCACTTCTGAGTTCGG3' of a DNA probe or the base sequence of 5'GUUUCACUUCUGAGUUCGG3' of an RNA probe, which are complementary only to the ribosomal RNA of Gram-negative bacteria such as Escherichia coli, Enterobacter cloacae, Proteus vulgaris, Proteus mirabilis, Proteus shigelloides, Serratia marcescens, Yersinia pestis, Salmonella typhimurium, Pseudomonas aeruginosa, Pseudomonas fluorescens, Acinetobacter calcoaceticus, Aeromonas hydrophila and Klebsiella pneumoniae, and the base sequence of 5'AGCTTAACTTCTGTGTTCGGCATGG3' of a DNA probe or the base sequence of 5'AGCUUAACUUCUGUGUUCGGCAUGG3' of an RNA probe, which are complementary only to the ribosomal RNA of Gram-positive bacteria such as Staphylococcus aureus, Staphylococcus epidermidis, and Streptococcus faecalis. As the probe, a DNA or RNA containing a base sequence of at least 12 successive bases in the above base sequence, which is labelled with a marker, may be used. In cases where bacteria are to be detected, the above-described probe for Gram-negative bacteria and for Gram-positive bacteria may be admixed and used.

Although both DNA and RNA may be used as the probe, DNA probe is preferred in view of its stability as a reagent.

The labelling substance used in the present invention includes markers _per se_ such as fluorescent substances, chemiluminescent substances, radioisotopes and enzymes, as well as those substances such as ligands and haptens including biotin, dinitrophenyl group and sulfonyl group, which can bind to the markers via avidin or an antibody.

Labelling of the DNA or RNA of the present invention containing the complementary base sequence with a labelling substance may be conducted by a conventional method such as nick translation method, photobiotin method and a method in which the end of the DNA or RNA is labelled with $^{32}P$ using polinucleotidekinase.

The sample used in the present invention is a sample derived from a living organism such as human, animals and plants, for which assaying of bacteria is required. Examples of the sample include blood, piercing fluid, urine, feces, pus, phlegm, abrasion product of mucosa, and foods and drinks such as milk and meat. The term "piercing fluid" means the fluid collected by piercing a needle in the body, such as pectoral fluid, ascites, articulation fluid and cerebrospinal fluid.

Assaying of bacteria using the probe of the present invention may be, for example, conducted as follows. The characteristic feature of the method of detecting bacteria of the present invention resides in that the method applies a molecular biological technique.

Firstly, the cells (such as erythrocytes and leucocytes) contained in a sample collected from the body are lysed. To lyse the cells, any of the general techniques such as changing pH or ionic strength, and addition of a surface active agent may be used. However, to shorten the lysing operation and not to increase very much the amount of the fluid after the lysis, it is preferred to use a surface active agent such as sodium dodecyl sulfate. By this operation, part of the ribosomal RNAs in the bacteria are eluted. However, in order to elute most of the ribosomal RNA, it is preferred to further extract the ribosomal RNAs with hot phenol. Further, in cases of Gram-positive bacteria, it is preferred to treat the bacteria with a lytic enzyme before the extraction with hot phenol. The eluted ribosomal RNA may be fixed to a membrane such as nitrocellulose and Nylon, or to polymer particles. Fixation may be conducted by means of the physical adsorption, ionic bond and covalent bond. In cases where the RNA is fixed to a membrane by means of physical adsorption, it is preferred to make sure that the RNAs on the membrane are not released in the subsequent steps by baking the membrane after fixation at about $80^{\circ}$C.

Excess amount of the above-described DNA or RNA probe containing a base sequence complementary to the base sequence of a bacterial ribosomal RNA is hybridized with the free or fixed bacterial ribosomal RNA derived from the sample as mentioned above. The remaining free probe, which did not hybridize may be removed. When the ribosomal RNAs are fixed to a membrane, the free probe may be removed by washing. In case of reaction in a liquid, the free probes may be separated by electrophoresis or the like.

After separating the free probes, the amount of the labelling substance of the probe which hybridized is determined. In cases where the labelling substance is an isotope, the amount may be determined by using a scintillation counter or a Geiger counter, or by exposing a film. In cases where the labelling substance is biotin or a hapten, avidin or an antibody to which an enzyme is fixed is further reacted with the probe, and thereafter the activity of the enzyme may be measured to determine the amount of the ribosomal RNA.

The characteristic feature of the present invention is as follows:

(1) Any kind of bacteria may be assayed by using only one or several probes. Furthermore, the bacteria population may be determined by quantifying the marker. (2) Since the ribosomal RNA is detected, not only live bacteria but also dead bacteria may be detected. Further, if a bacterium is damaged by an operation for destroying phagocytes or the like, the bacterium may be detected. Since a phagocyte eats a number of bacteria, if the bacteria in the phagocyte can be detected, the amount of the sample may be reduced. (3) Ribosomes contained in bacteria in a great amount are detected. Although the amount of the ribosomes varies depending on the conditions, the usual content is $10^3$ to $10^4$ copies/cell. Therefore, the detection sensitivity of the method of the present invention may be made high. (4) Since the time required for the detection is shortened, it is easy to utilize the results of the assay in a therapy.

The present invention will now be described in more detail by way of examples.

Example 1

Detection of 16 S and 23 S RNA in Escherichia coli

A. Fixation of E. coli Ribosomal RNAs to Membranes

16 S and 23 S ribosomal RNAs of E. coli (Boehringer-Mannheim GmbH) were serially 10-fold diluted with TE buffer (10 mM Tris-HCl, 1 mM EDTA, pH8.0). Each of them was adsorbed to a cationic Nylon membrane (Pall "Biodyne B") using a suction filtration apparatus ("Minifold" of Schleicher & Schuell). The membranes were baked at $80^O$C to fix the ribosomal RNAs.

B. Preparation of DNA Probe Complementary to Ribosomal RNAs

A plasmid containing genes coding for 5 S, 16S and 23 S ribosomal RNAs (hereinafter referred to as pKK) was prepared in accordance with the method of J. Brosius et

al (J. Mol. Biol., 148, 107-127 (1981)).  The base number of the plasmid pKK was about 11.9 kb.  The pKK was subjected to the nick translation at 15°C for 60 minutes with 2.5 ng/ml of DNase I (Pharmacia, AB) and 200 U/ml of DNA polymerase I (Takara Shuzo Co., Ltd.) in a solution containing 50 mM Tris-HCl (pH7.2), 10 mM of magnesium chloride, 0.1 mM of dithiothreitol, 50 μg/ml of bovine serum albumin (BSA), 20 μM each of dATP, dGTP and dTTP, and 2 μM of $[\alpha-^{32}P]$dCTP (Amersham International, PLC) to label the pKK with $^{32}$P.  The specific activity was $10^7$ cpm/μg.

C.    Detection of Ribosomal RNAs by Hybridization

The membranes were incubated at 42°C for 3 hours in a hybridization solution containing 5 x SSPE (100 mM sodium phosphate buffer (pH7.8) containing 0.9 M of NaCl and 5 mM of EDTA), 0.1% of sodium dodecyl sulfate (SDS), 40% of formamide, 0.1 g/ml of BSA, 0.1 g/ml of Ficoll, 0.1 g/ml of polyvinyl pyrrolidone and 0.1 mg/ml of thermally denatured salmon sperm DNAs.  Thereafter, the membranes were transferred into a fresh hybridization medium.  Thermally denatured $^{32}$P-labelled DNA probe was added to the solution to a final concentration of $10^6$ cpm/ml, and the solution was incubated at 42°C overnight. The membranes were washed three times with 2 x SSC (30 mM of sodium citrate containing 0.3 M of NaCl) containing 0.1% SDS at 42°C for 20 minutes.  The membranes were air-dried and were subjected to autoradiography at -70°C.

As a result, the detection limit was 2.6 x $10^{-15}$ mol in case of 22 hours exposure.

Example 2

Detection of Bacteria

A.    Fixation of Bacterial Ribosomal RNAs to Membranes

Six strains of Gram-negative bacteria, i.e., E. coli (JCM1649), Enterobacter cloacae (JCM1232), Klebsiella pneumoniae (JCM1662), Proteus vulgaris (JCM1668), Serratia marcescens (JCM1239) and Pseudomonas aeruginosa

(JCM2776), and 2 strains of Gram-positive bacteria, i.e., Staphylococcus aureus (JCM2413) and Streptococcus faecalis (JCM2875), all strains being obtained from Rikagaku Kenkyusho, were cultured overnight. Each strain was suspended in 1 ml of water at a density of $10^9$ cells/ml, the suspension was centrifuged (10,000 rpm, 5 minutes, hereinafter these conditions), and the supernatant was discarded. The remaining bacterial cells were suspended in about 20 μl of water. To the suspension, were added 5 μl of lytic enzyme solution (0.5 mg/ml of N-acetylmuramidase, 0.5 mg/ml of lysopeptidase, 10 mM Tris-HCl, pH8.0), and the suspension was incubated at 37°C for 15 minutes. The suspension was then suspended in 400 μl of TE buffer. The resulting suspension was mixed with 400 μl of TE buffer-saturated phenol and the mixture was incubated at 65°C for 15 minutes. After centrifugation, aqueous layer (upper layer) was separately taken and the remaining phenol was removed by using diethyl ether. This was serially 10-fold diluted with water and the dilutions were adsorbed to Nylon membranes as in Example 1.

B.    Preparation of DNA probe Complementary to Ribosomal RNA

The procedures described in Example 1 were followed. The specific activity was $10^8$ cpm/μg.

C.    Detection of Ribosomal RNAs by Hybridization

The procedures described in Example 1 were followed. The results are shown in Table 1.

Table 1

| | | Detection Sensitivity | |
|---|---|---|---|
| | | 2 Hours Exposure | Overnight Exposure |
| Gram-negative Bacteria (6 strains) | E. coli | $10^4$ cells | $10^3$ cells |
| | Enterobacter cloacae | ditto | ditto |
| | Klebsiella pneumoniae | ditto | ditto |
| | Proteus vulgaris | ditto | ditto |
| | Serratia marcescens | ditto | ditto |
| | Pseudomonas aeruginosa | ditto | ditto |
| Gram-Positive Bacteria (2 strains) | Staphylococcus aureus | $10^5$ cells | $10^4$ cells |
| | Streptococcus faecalis | ditto | ditto |

Example 3

Detection of E. coli in the Blood

A.  Fixation of Samples to Membranes

To 1 ml of human whole blood or to 1 ml of water, $10^7$ or $10^6$ cells of E. coli were added.  To each of these, 0.1% of Triton X-100 was added to hemolyze the blood and the mixture was centrifuged.  After removing the supernatant, this procedure was repeated once again. Thereafter, bacteriolysis, enzyme treatment, phenol treatment and ether treatment were conducted in the same manner as in Example 2A.  The samples were adsorbed and fixed to Nylon membranes.

After hybridization as in Example 2B, E. coli was detected by exposing the film for 2 hours.

As a result, even if the cells of E. coli were added to the whole blood, E. coli was detected in the same degree of sensitivity as in the case where the cells were added to water.

On the other hand, while whole blood was treated in the same manner, hybrids were not formed.

Example 4

Identification of Gram-negative Bacteria and Gram-positive Bacteria by DNA Oligomer

A.     Preparation of Bacterial Ribosomal RNA

About 4 $\mu$l aliquot (corresponding to $10^7$ cells) of the aqueous solution of ribosomal RNAs treated as in Example 2 was used in C.

B.     Preparation of DNA Probe

DNA probe 1 (5'GTTTCACTTCTGAGTTCGG) complementary to the ribosomal RNA of Gram-negative bacteria and DNA probe 2 (5'AGCTTAACTTCTGTGTTCG) complementary to the ribosomal RNA of Gram-positive bacteria were treated with 500 U/ml of T4 polynucleotide kinase (Takara Shuzo, Co., Ltd.) in a solution containing 70 mM Tris-HCl (pH7.6), 10 mM magnesium chloride, 5 mM dithiothreitol and 1 $\mu$M of ($\gamma$-$^{32}$P)ATP (Amersham International, PLC) at 37$^{\circ}$C for 30 minutes to label the 5' end of the DNAs with $^{32}$P.

C.     Detection of Hybrids

A 10 $\mu$l solution containing $10^7$ bacterial cells prepared in A, $10^4$ cpm of the probe, and NaCl (0.5 M for probe 1 and 0.05 M for probe 2) was incubated at 100$^{\circ}$C for 10 minutes, and then at 60$^{\circ}$C for 20 minutes and cooled in ice to allow the formation of hybrids. Thereafter the solution was subjected to 15% acrylamide gel electrophoresis to separate the hybrids from the free probes.  The gel was exposed at -70$^{\circ}$C overnight.

The results are shown in Table 2.

- 14 -                                          0277237

Table 2

| | | Formation of Hybrids | |
| | | Probe 1 | Probe 2 |
|---|---|---|---|
| Gram-negative Bacteria (6 strains) | E. coli | + | - |
| | Enterobacter cloacae | + | - |
| | Klebsiella pneumoniae | + | - |
| | Proteus vulgaris | + | - |
| | Serratia marcescens | + | - |
| | Pseudomonas aeruginosa | + | - |
| Gram-Positive Bacteria (2 strains) | Staphylococcus aureus | - | + |
| | Streptococcus faecalis | - | + |

Example 5

Detection of Bacteria in Blood

To 1 ml of human whole blood, $10^9$ cells each of E. coli, Pseudomonas aeruginosa or Staphylococcus aureus were added, and the mixtures were treated as in Example 3. Four microliters aliquots of the mixtures were used for the hybridization to obtain the same results as in the buffer system, which are shown in Table 3.

Table 3

| | Formation of Hybrids | |
| | Probe 1 | Probe 2 |
|---|---|---|
| E. coli Added to Whole Blood | + | - |
| P. aeruginosa Added to Whole Blood | + | - |
| S. aureus Added to Whole Blood | - | + |
| Whole Blood Containing No Bacteria | - | - |

INDUSTIAL APPLICABILITY

The assaying method and the probe of the present invention are very useful in detecting bacteria contained in a sample such as blood, piercing fluid and urine since (1) dead bacteria and bacteria eaten by phagocytes can be detected, (2) the time required for the assay is shortened from several days to 1 day or less, (3) various kinds of bacteria can be assayed simultaneously, and (4) detection sensitivity is high because the ribosomal RNAs contained in a large amount in the bacterial cells are detected, so that the amount of the samples may be reduced, and so on.

CLAIMS

1. A method of assaying bacteria in a sample by using a probe prepared by labelling a DNA or RNA with a labelling substance, which DNA or RNA contains a base sequence complementary to a base sequence of at least 12 successive bases of a ribosomal RNA of Escherichia coli.

2. The method of assaying bacteria of claim 1, wherein the DNA or RNA is complementary only to the ribosomal RNA of Gram-negative bacteria or only to the ribosomal RNA of Gram-positive bacteria.

3. The method of assaying bacteria of claim 2, wherein the DNA or RNA complementary only to the ribosomal RNA of Gram-negative bacteria has a base sequence of at least 12 successive bases in a base sequence of GTTTCACTTCTGAGTTCGG or GUUUCACUUCUGAGUUCGG.

4. The method of assaying bacteria of claim 2, wherein the DNA or RNA complementary only to the ribosomal RNA of Gram-positive bacteria has a base sequence of at least 12 successive bases in a base sequence of AGCTTAACTTCTGTGTTCGGCATGG or AGCUUAACUUCUGUGUUCGGCAUGG.

5. A probe prepared by labelling a DNA or RNA with a labelling substance, which DNA or RNA contains a base sequence complementary to a base sequence of at least 12 successive bases of a ribosomal RNA of Escherichia coli.

6. The probe of claim 5, wherein the DNA or RNA is complementary only to the ribosomal RNA of Gram-negative bacteria or only to the ribosomal RNA of Gram-positive bacteria.

7. The probe of claim 6, wherein the DNA or RNA complementary only to the ribosomal RNA of Gram-negative bacteria has a base sequence of at least 12 successive bases in a base sequence of GTTTCACTTCTGAGTTCGG or GUUUCACUUCUGAGUUCGG.

8. The probe of claim 6, wherein the DNA or RNA complementary only to the ribosomal RNA of Gram-positive bacteria has a base sequence of at least 12 successive bases in a base sequence of AGCTTAACTTCTGTGTTCGGCATGG or AGCUUAACUUCUGUGUUCGGCAUGG.

0277237

# INTERNATIONAL SEARCH REPORT

International Application No PCT/JP87/00486

## I. CLASSIFICATION OF SUBJECT MATTER (if several classification symbols apply. indicate all) 3

According to International Patent Classification (IPC) or to both National Classification and IPC

Int.Cl$^4$ C12Q1/68, C12N15/00 //C12Q1/04

## II. FIELDS SEARCHED

Minimum Documentation Searched 4

| Classification System | Classification Symbols |
|---|---|
| IPC | C12Q1/68, C12Q1/04, C12N15/00 |

Documentation Searched other than Minimum Documentation
to the Extent that such Documents are Included in the Fields Searched 5

## III. DOCUMENTS CONSIDERED TO BE RELEVANT 14

| Category* | Citation of Document, 16 with indication, where appropriate, of the relevant passages 17 | Relevant to Claim No. 18 |
|---|---|---|
| X | JP, A, 60-100056 (Miles Laboratories Inc.) 3 June 1985 (03. 06. 85) & EP, A2, 133671 | 1-2, 5-6 |
| X | JP, A, 60-500895 (Webster John E., Jr.) 20 June 1985 (20. 06. 85) & WO, A1, 8403715 & WO, A1, 8403716 & EP, A2, 120658 | 1-2, 5-6 |
| X | JP, A, 60-501339 (Jen-Probe Partners) 22 August 1985 (22. 08. 85) & WO, A1, 8402721 & EP, A1, 131052 | 1-2, 5-6 |
| X | JP, A, 58-501496 (Webster John E., Jr.) 8 September 1983 (08. 09. 83) & WO, A1, 8301073 & EP, A1, 76123 & EP, A1, 155359 & EP, A1, 155360 | 1-2, 5-6 |

* Special categories of cited documents: 15

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure. use. exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents. such combination being obvious to a person skilled in the art

"&" document member of the same patent family

## IV. CERTIFICATION

Date of the Actual Completion of the International Search :

September 22, 1987 (22. 09. 87)

Date of Mailing of this International Search Report :

October 5, 1987 (05. 10. 87)

International Searching Authority 1

Japanese Patent Office

Signature of Authorized Officer 20

Form PCT/ISA/210 (second sheet) (October 1981)